(19) **Europäisches Patentamt**
**European Patent Office**
**Office européen des brevets**

(11) **EP 2 732 808 B1**

(12) **EUROPEAN PATENT SPECIFICATION**

(45) Date of publication and mention
of the grant of the patent:
**13.03.2019 Bulletin 2019/11**

(21) Application number: **12811868.4**

(22) Date of filing: **06.07.2012**

(51) Int Cl.:
*A61K 8/90* *(2006.01)*     *A61K 8/37* *(2006.01)*
*A61K 8/39* *(2006.01)*     *A61K 8/86* *(2006.01)*
*A61Q 1/14* *(2006.01)*     *A61Q 5/06* *(2006.01)*
*A61Q 19/00* *(2006.01)*

(86) International application number:
**PCT/JP2012/067385**

(87) International publication number:
**WO 2013/008758 (17.01.2013 Gazette 2013/03)**

(54) **COSMETIC COMPOSITION**

KOSMETISCHE ZUSAMMENSETZUNG

COMPOSITION COSMÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.07.2011 JP 2011152690**

(43) Date of publication of application:
**21.05.2014 Bulletin 2014/21**

(73) Proprietor: **NOF Corporation
Shibuya-ku
Tokyo 150-6019 (JP)**

(72) Inventors:
• **TEZUKA Yoji
Kawasaki-ku, Kawasaki-shi
Kanagawa 210-0865 (JP)**

• **IIZUKA Muneaki
Kawasaki-ku, Kawasaki-shi
Kanagawa 210-0865 (JP)**

(74) Representative: **Hoffmann Eitle
Patent- und Rechtsanwälte PartmbB
Arabellastraße 30
81925 München (DE)**

(56) References cited:
**WO-A1-2006/038724     JP-A- 2006 282 540
JP-A- 2007 217 392      JP-A- 2007 269 719
JP-A- 2008 088 127      JP-A- 2009 227 646
JP-A- 2010 024 159      JP-A- 2012 017 305
US-A1- 2006 078 525     US-A1- 2009 163 616
US-A1- 2010 075 882     US-A1- 2010 190 864**

**Description**

Technical Field

The present invention relates to a cosmetic composition containing a polyalkylene glycol derivative and a nonionic surfactant.

Background Art

An oily agent in cosmetics is an important constitutional component which decides appearance and a feeling in use. Therefore, an oily agent that is a transparent liquid at ordinary temperature, exhibits no stickiness, and has a good spreadability has been desired.

Examples of transparent cosmetics highly blended with an oily agent may include cleansing oils, hair oils, massage oils, and the like. An excellent structure-recovering property may be mentioned as a consumer's need for these cosmetics in recent years.

The structure-recovering property is a property that viscosity decreases by the action of an external force but returns to the original one when the external force is suppressed. In general, an oily agent exhibiting a good feeling in use has a low viscosity and causes liquid dripping when taken in the hand, and thus it is difficult to apply it to a suitable part of the skin in a proper quantity. Therefore, it is required to have a high viscosity at the time when it is taken out of a vessel or before it is applied to the skin. On the other hand, at the time when it is applied to the skin (an external force is imparted), it is required not to impair the smoothness intrinsic to the oily agent, that is, when a feeling in use is good and the structure-recovering property is exhibited, added value of commercial products can be enhanced.

In order to impart the structure-recovering property, it is common to add a gelling agent or thickening agent (hereinafter referred to as gelling agent) to control the viscosity, but it is technically difficult to obtain an oily gel. The reason is that it is difficult to form an association by the action of electric charge since electric conductivity is poor in an oily system unlike the case in an aqueous system and further oily agents include various chemical substances such as hydrocarbon oils, vegetable oils and glycerides, ester oils, and silicone oils and thus their thermodynamic properties are variously different. Therefore, it is difficult to prepare an oily gel that exhibits no temperature dependency and has a good thermal stability.

Namely, it has been desired to develop a cosmetic composition which has the structure-recovering property, is transparent, has a good thermal stability, and exhibits a good feeling in use, with containing a large amount of an oily agent.

In order to enhance the viscosity of the oily agent, some gelling agents have been hitherto proposed. For example, a technology using 12-hydroxystearic acid and a dextrin fatty acid such as dextrin palmitate (Patent Document 1), an N-acylamino acid derivative such as N-2-lauroyl-L-glutamic acid dibutylamide (Patent Document 2), and the like have been proposed. They show a gelling mechanism resulting from a hydrogen bond which strongly interacts even in an oil, and exhibit a gelling effect in a small amount and are excellent in thermal stability, but they are not proposals for the purpose of achieving the structure-recovering property and an excellent feeling in use when applied to the skin and hair. Moreover, there has been also proposed a gel composition containing a specific polyoxyalkylene ether that is a block-type alkylene oxide derivative, an oily agent, and water (Patent Document 3). The composition has a gel-forming ability having an excellent structure-recovering property (thixotropic property) and a transparent feeling, but there is a further room for improvement in a feeling in use, for example, a feeling and spreadability when applied.

Therefore, it has been desired to develop a cosmetic composition which has the structure-recovering property, is transparent, has a good thermal stability, and also exhibits a good feeling in use.

Patent Document 4 describes a fine emulsion composition containing a block type alkylene oxide derivative having a specific formula, an oily component, a polyol and water. Patent Document 5 describes an alkylene oxide derivative having a specific formula which is used in a skin external preparation. Patent document 6 describes a gel composition comprising a specific block-type alkylene oxide derivative, an oil and water which can easily form an oil-based gel with a high stability.

Prior Art Documents

Patent Documents

Patent Document 1: JP-A-2002-3340
Patent Document 2: JP-A-2002-316971

Patent Document 3: JP-A-2008-19239
Patent Document 4: JP-A-2010-024159
Patent Document 5: US 2010/0190864 A1
Patent Document 6: US 2009/0163616 A1

Summary of the Invention

Problems that the Invention is to Solve

A problem that the invention is to solve is to provide a cosmetic composition which has a structure-recovering property, is transparent, is excellent in thermal stability, and has an extremely good feeling in use, for example, smoothness when applied to the skin or hair, fitness with the skin, and the like.

Means for Solving the Problems

As a result of extensive studies, the present inventors have found that the above problem is solved by blending a specific polyalkylene glycol derivative and a specific nonionic surfactant.
Namely, the present invention lies on the following.

(1) A cosmetic composition comprising the following components (A) to (D), wherein the component (A) is from 0.1 to 30% by mass, the component (B) is from 0.1 to 30% by mass, the component (C) is from 50 to 95% by mass wherein a part of component (C) can be replaced by the component (E) and an amount of the component (C) is 30 parts by mass or more relative to 100 parts by mass of a total amount of the component (C) and the component (E), and the component (D) is from 0.01 to 10% by mass, and a mass ratio of the component (A) to the component (B) wherein the amounts of the components (A) to (D) add up to 100% by mass, (A)/(B) is from 1/5 to 5/1:

(A) a polyalkylene glycol derivative represented by the following formula (I):

$$Z\text{-}\{O\text{-}(EO)_a\text{-}(AO)_k\text{-}H\}_m \qquad (I)$$

wherein Z is a residual group resulting from removal of hydroxyl groups from a polyhydric alcohol having 3 to 6 hydroxyl groups, m is from 3 to 6, EO is an oxyethylene group, AO is an oxyalkylene group having 4 to 8 carbon atoms, and EO and AO are bonded in a block form;
a represents an average addition mole number of EO and k represents an average addition mole number of AO, and a is from 1 to 50 and k is from 1 to 50; and a mass ratio of EO is from 10 to 75 parts by mass relative to 100 parts by mass of a total amount of EO and AO,

(B) a nonionic surfactant having a hydroxyl group and a hydrocarbon group having 12 to 22 carbon atoms and exhibiting an HLB of 3 to 8,
(C) one or two or more oily agents selected from ester oils and triglycerides which are liquid at 25°C, and
(D) water.
(E) one or two or more oily agents selected from hydrocarbon oils and silicone oils which are liquid at 25°C.

(2) The cosmetic composition according to (1), wherein AO is a 1,2-oxybutylene group.
(3) The cosmetic composition according to (1) or (2), wherein the component (B) is a nonionic surfactant having a branched or unsaturated hydrocarbon group having 12 to 22 carbon atoms.

Advantage of the Invention

The cosmetic composition of the invention can achieve both of a transparent appearance and a structure-recovering property and is also excellent in thermal stability although the composition has a composition containing a large amount of oily components. That is, since the composition has an excellent structure-recovering property, liquid dripping is not observed at the time when it is taken out of a vessel and a proper quantity thereof can be taken in the hand without smearing the vessel. When it is applied to the skin and an external force is imparted, for example, spreading it by a fingertip, the viscosity decreases and a smooth feeling intrinsic to an oily preparation can be obtained, so that a feeling in use, such as fitness with the skin or hair, is also extremely good.

Brief Description of the Drawings

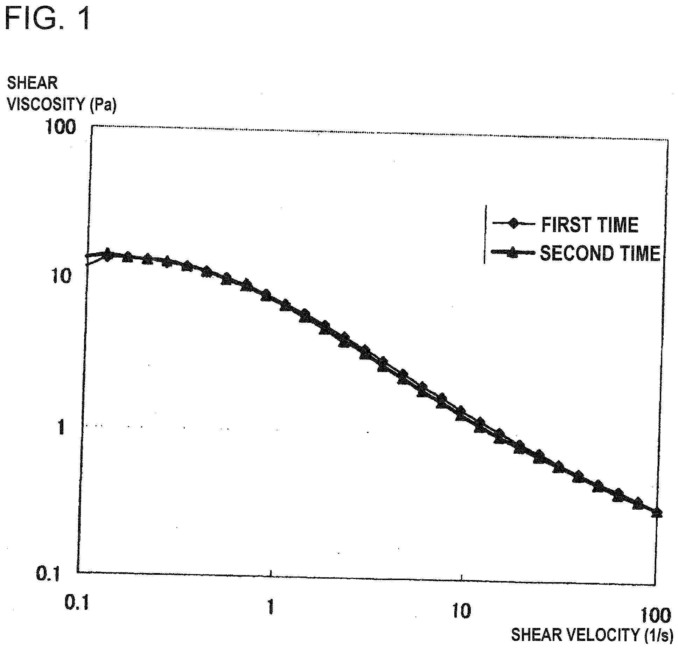

Fig. 1 is a drawing showing a relationship between shear velocity and shear viscosity of the cosmetic composition of Example 1 (structure-recovering property: ○, recovering ratio: 115%) .

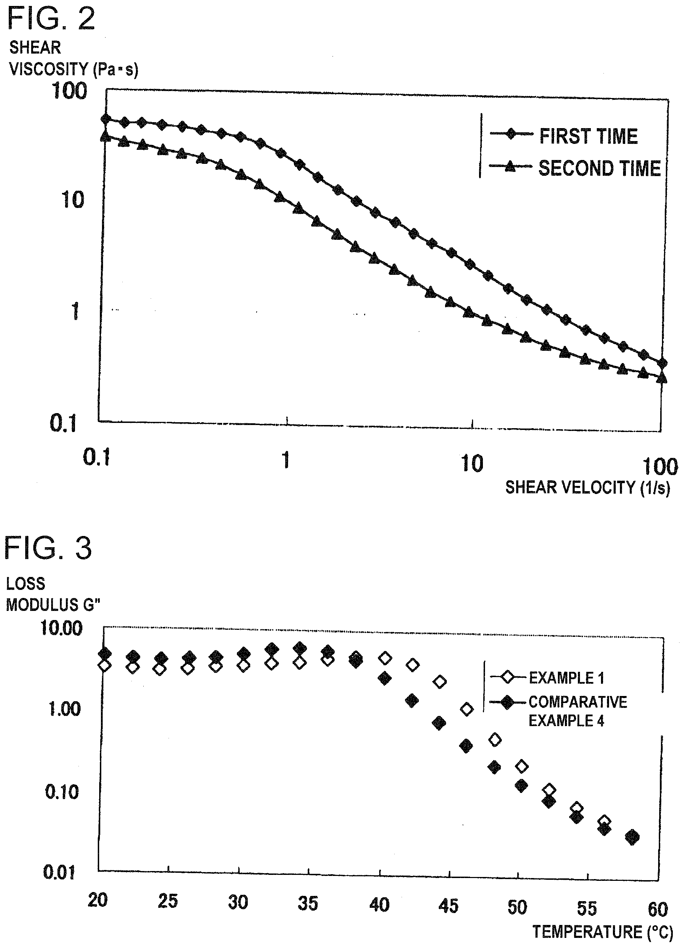

Fig. 2 is a drawing showing a relationship between shear velocity and shear viscosity of the cosmetic composition of Comparative Example 13 (structure-recovering property: ×, recovering ratio: 65%).
Fig. 3 is a drawing showing a relationship between temperature and loss modulus G" of Example 1 (thermal stability: ○) and Comparative Example 4 (thermal stability: △).

Mode for Carrying Out the Invention

The component (A) is a polyalkylene glycol derivative represented by the following formula (I):

$$Z\text{-}\{O\text{-}(EO)_a\text{-}(AO)_k\text{-}H\}_m \qquad (I)$$

wherein Z is a residual group resulting from removal of hydroxyl groups from a polyhydric alcohol having 3 to 6 hydroxyl groups. Examples of the polyhydric alcohol include glycerin and trimethylolpropane having three hydroxyl groups, diglycerin, erythritol, pentaerythritol, sorbitan, and methylglycoside having four hydroxyl groups, xylitol and triglycerin having five hydroxyl groups, and sorbitol, inositol, and dipentaerythritol having six hydroxyl groups. Preferred are residual groups obtained by removing hydroxyl groups from alcohols having four or more hydroxyl groups and, owing to a high gelling ability, more preferred are diglycerin and pentaerythritol having four hydroxyl groups.
m is from 3 to 6, preferably from 4 to 6, more preferably 4. When m is 2 or less, thermal stability is not sufficient and a stickiness is generated when applied and thus a smooth feeling in use is not obtained, so that the case is not preferred. When m is 7 or more, a sticky feeling is generated when applied and a smooth feeling in use is also not obtained, so that the case is not preferred.

EO is an oxyethylene group, a represents an average addition mole number of EO, and $a \times m$ is a total addition mole number of EO in the formula (I). a is from 1 to 50, preferably from 10 to 45. When a is smaller than 1, a sufficient hydrophilicity is not obtained and a cosmetic composition having a structure-recovering property is not obtained. Moreover, when a is larger than 50, crystallinity of the EO chain becomes high, so that a transparent cosmetic composition is not obtained.

$(EO)_a$ is a polyoxyethylene chain and becomes a hydrophilic group in the polyalkylene glycol derivative of the invention.

AO is an oxyalkylene group having 4 to 8 carbon atoms, and the AO chain becomes a lipophilic group in the polyalkylene glycol derivative of the formula (I). Since the AO chain contributes an improvement in compatibility between the component (A) and the component (C) and/or the component (E), the chain is an essential part for retaining the stability of the cosmetic composition.

In the case where AO has 3 carbon atoms, the compatibility with the oily agent is poor and desired thickening ability is not obtained. Furthermore, in the case where AO has 9 or more carbon atoms, the feeling in use tends to become worse, so that the case is not preferred.

Examples of AO include a 1,2-oxybutylene group having 4 carbon atoms, a 1,2-oxypentylene group having 5 carbon atoms, a 1,2-oxyhexylene group having 6 carbon atoms, a 1,2-oxyheptylene group having 7 carbon atoms, and a 1,2-oxyoctylene group having 8 carbon atoms, but preferred is a 1,2-oxybutylene group or a 1,2-oxyoctylene group and more preferred is a 1,2-oxybutylene group. AO may be one kind or two kinds or more and in the case where it is two kinds or more, the addition form may be any of a random form or a block form.

k represents an average addition mole number of AO, and $k \times m$ represents a total average addition mole number of AO in the formula (I). k is from 1 to 50, preferably from 5 to 30. When k is smaller than 1, a sufficient lipophilicity is not obtained and the compatibility between the component (A) and the component (C) and/or the component (E) is poor, so that a stable composition is not obtained. Moreover, when k is larger than 50, a sticky feeling is generated, so that there is a case where a feeling in use is poor and hence the case is not preferred.

When the total content of EO and AO is taken as 100 parts by mass, the mass ratio of EO is from 10 to 75 parts by mass. Preferred is from 20 to 75 parts by mass. More preferred is from 30 to 75 parts by mass. When the mass ratio is smaller than 10 parts by mass, a hydrated region decreases and gelation does not take place even when water of the component (D) is added. Moreover, when the mass ratio is larger than 75 parts by mass, the ratio of the EO chain becomes large, so that crystallinity increases and hence a transparent cosmetic composition is not obtained.

EO and AO are bonded in a block form. In a random form, a desired oily cosmetic composition cannot be obtained.

The polyoxyalkylene glycol derivative represented by the formula (I) of the invention can be produced by known methods. For example, the derivative can be obtained by addition polymerization of a polyhydric alcohol having 3 to 6 hydroxyl groups with oxyethylene and an oxyalkylene having 4 to 8 carbon atoms in this order under an alkali or acid catalyst.

The component (A) is blended in an amount of 0.01 to 30% by mass relative to the total mass of the cosmetic composition. The amount is preferably from 0.1 to 20% by mass, more preferably from 1 to 15% by mass. When the amount is less than 0.01% by mass, the desired structure-recovering property is not obtained. When the amount exceeds 30% by mass, a sticky feeling is generated when the composition is applied and thus the case is not preferred.

The component (B) is a nonionic surfactant having a hydroxyl group and a hydrocarbon group having 12 to 22 carbon atoms and exhibiting an HLB of 3 to 8. Incidentally, the nonionic surfactant is sometimes singly referred to as nonion.

HLB is an abbreviation of Hydrophile-Lipophile Balance and is a concept resulting from numeric conversion of the balance between the hydrophilic group and the lipophilic group of a surfactant. In general, it is represented in the range of 0 to 20 and a higher numerical value shows higher hydrophilicity. HLB can be calculated from the following formula (1) or (2).

(1) Polyglycerin-type or sorbitan nonionic surfactant

$$HLB = 20(1-S/A)$$

S: saponification value of ester, A: acid value of fatty acid
(Source: "Shin-pan Kaimen Kasseizai Handobukku (New Edition Surfactant Handbook)" Kougaku Tosho K.K.)

(2) Glucide-type nonionic surfactant

$$HLB = (Sum\ of\ inorganicity\ value/Sum\ of\ organicity\ value) \times 10$$

Inorganicity value: Hydroxyl group = 100,
Ether bond = 20,
Cyclic ether bond of sugar = 75
Organicity value: Carbon atom = 20, Iso branching = -10 (Source: "Yuuki Gainen-zu - Kiso to Ouyo (Organic Conceptual Diagram - Base and Application)" written by Kouda, Sankyo Publishing Co., Ltd., "Yuuki Ginen-zu niyoru Nyuuka Syohou Sekkei (Emulsification Formulation Design by Organic Conceptual Diagram)" written by Yaguchi, Nihon Emulsion Co., Ltd.)

In the case of the nonion exhibiting an HLB of smaller than 3, since hydroxyl group concentration in the molecule is low, swelling by hydration is insufficient even when water of the component (D) is added and the desired structure-recovering property is not obtained. When HLB exceeds 8, hydration with water is strong and the composition tends to coagulate by the influence of the EO chain of the component (A), so that a sticky feeling is generated when applied and hence the case is not preferred.

Examples of the hydrocarbon group having 12 to 22 carbon atoms include a lauryl group, a tridecyl group, a myristyl group, a palmityl group, a cetyl group, an isopalmityl group, a stearyl group, an isostearyl group, an oleyl group, an octyldodecyl group, a vehenyl group, and the like and the hydrocarbon group may be a mixed alkyl group thereof. Of these, from the viewpoints of compatibility and transparency, branched saturated hydrocarbon groups or unsaturated hydrocarbon groups having 12 to 22 carbon atoms are preferred. Particularly preferred are an oleyl group, an isopalmityl group, and an isostearyl group. When the carbon number is smaller than 12, a desired structure-recovering property is not obtained and thus the case is not preferred. Also, when the carbon number exceeds 22, transparency is not obtained.

Examples of the hydroxyl group of the component (B) may include hydroxyl groups derived from polyhydric alcohols such as glycerin, polyglycerin, sorbitol, and sorbitan and hydroxyl groups derived from sugars such as glucose, sucrose, and trehalose, but preferred are hydroxyl groups derived from glycerin, polyglycerin having a polymerization degree of 2 to 6, or sorbitan for achieving transparency and the structure-recovering property. Moreover, in order to enhance hydration with water of the component (D), the component (B) is preferably an ester-type nonion. For instance, examples of polyhydric alcohol fatty acid ester-type and sugar fatty acid ester-type nonionic surfactants include sorbitan monooleate ester, sorbitan sesquioleate ester, sorbitan dioleate ester, sorbitan trioleate ester, sorbitan monoisostearate ester, sorbitan sesquiisostearate ester, sorbitan diisostearate ester, sorbitan triisostearate ester, glycerin monooleate ester, polyglycerin oleate ester, glycerin monoisostearate ester, polyglycerin isostearate ester, sucrose oleate ester, sucrose palmitate ester, and the like. More preferred are sorbitan oleate ester, sorbitan sesquioleate ester, sorbitan isostearate ester, sorbitan sesquiisostearate ester, diglycerin dioleate ester, and diglycerin diisostearate ester. Moreover, an EO addition-type nonion is not preferred since the thermal stability is not sufficient.

The component (B) is blended in an amount of 0.1 to 30% by mass relative to the total mass of the cosmetic composition. The amount is preferably from 1 to 20% by mass. When the amount is less than 0.1% by mass, the desired structure-recovering property is not obtained. When the amount exceeds 30% by mass, transparency cannot be maintained and thus the case is not preferred. The component (B) may be used with mixing two or more kinds thereof. Moreover, the mass ratio of the component (A) to the component (B), (A)/(B) is from 1/5 to 5/1, preferably from 1/3 to 3/1.

The component (C) is one or two or more oily agents selected from ester oils and triglycerides which are liquid at 25°C.

Examples of the ester oils include ethyl oleate, ethyl linoleate, isopropyl myristate, isopropyl palmitate, isopropyl isostearate, cetyl 2-ethylhexanoate, isocetyl 2-ethylhexanoate, stearyl 2-ethylhexanoate, isostearyl 2-ethylhexanoate, cetyl palmitate, 2-ethylhexyl palmitate, 2-hexyldecyl isostearate, isostearyl isostearate, trimethylolpropane triisostearate, myristyl myristate, cetyl myristate, octyldodecyl myristate, isostearyl myristate, isocetyl myristate, hexyl laurate, decyl oleate, octyldodecyl oleate, isostearyl pivalate, isopropyl isostearate, isononyl isononanoate, 2-ethylhexyl isononanoate, isodecyl isononanoate, isotridecyl isononanoate, octyldodecyl erucate, neopentyl glycol didecanoate, pentaerythrityl tetraethylhexanoate, diisosteary malate, triethylhexanoate trimethylolpropane didecyl adipate, didecyl adipate, cholesteryl isostearate, batyl isostearate, hardened caster oil monohydroxystearate, lanolin fatty acid isostearyl ester, lanolin fatty acid isopropyl ester, lanolin fatty acid octyldodecyl ester, cetyl ricinoleate, dioctyl succinate, cetyl lactate, propylene glycol dicaprylate, propylene glycol dicaprate, propylene glycol dinonanoate, propylene glycol di(caprylate·caprate), propylene glycol diisostearate, propylene glycol dioleate, and the like. One kind or two or more kinds thereof may be used.

Examples of the triglycerides include triglycerides of glycerin with a higher fatty acid having 6 or more carbon atoms, such as caproic acid, caprylic acid, capric acid, 2-ethylhexanoic acid, isotridecanoic acid, isopalmitic acid, isostearic acid, eicosanoic acid, or oleic acid, animal and vegetable oils and fats such as olive oil, sunflower seed oil, safflower oil, castor oil, and camellia oil, and the like.

Preferable ester oils include cetyl 2-ethylhexanoate, isocetyl 2-ethylhexanoate, isopropyl palmitate, 2-ethylhexyl palmitate, isopropyl isostearate, 2-hexyldecyl isostearate, octyldodecyl myristate, isopropyl myristate, isostearyl myristate, isocetyl myristate, isononyl isononanoate, 2-ethylhexyl isononanoate, isodecyl isononanoate, and isotridecyl isononanoate.

Moreover, examples of preferable triglycerides include triglycerides of glycerin with a higher fatty acid having 6 to 10 carbon atoms, such as caproic acid, caprylic acid, capric acid, or 2-ethylhexanoic acid or a mixture thereof, for example, a glyceride of tri(caprylate/caprate).

In the cosmetic composition of the invention, a part of the component (C) can be replaced by the component (E).

The component (E) is one or two or more oily agents selected from hydrocarbon oils and silicone oils which are liquid at 25°C. The hydrocarbon oils include liquid paraffin, hydrogenated polyisobutene, hydrogenated polydecene, squalane, squalene, pristane, light isoparaffin, light liquid isoparaffin, heavy liquid isoparaffin, liquid isoparaffin, tetradecene, isohexadecane, isododecane, $\alpha$-olefin oligomers, and the like. Preferred are liquid paraffin, hydrogenated polyisobutene, and squalane. The silicone oils include dimethicone, cyclomethicone, phenyldimethicone, and the like, but preferred is dimethicone. Also, one kind or two or more kinds thereof may be used.

The component (C) and the component (E) are blended in an amount of 50 to 95% by mass as the total amount of both components relative to the total mass of the cosmetic composition. The total amount is preferably from 60 to 90% by mass. When the amount is less than 50% by mass, a feeling in use becomes worse, such as stickiness. Moreover, when the amount exceeds 95% by mass, viscosity cannot be imparted and thus the case is not preferred. Also, when the total amount of the component (C) and the component (E) is taken as 100 parts by mass, the amount of the component (C) is 30 parts by mass or more, preferably 40 parts by mass or more, more preferably 50 parts by mass or more. When the amount is less than 30 parts by mass, the desired structure-recovering property is not obtained and thus the case is not preferred.

The component (D) is water. In the cosmetic composition of the invention, viscosity is imparted by hydration of the EO chain of the component (A) or the hydroxyl group of nonion of the component (B). The water is not particularly limited as long as it is generally used in cosmetics, quasi drugs, and pharmaceuticals. For example, purified water such as distilled water or ion-exchanged water, physiological saline, phosphate buffer aqueous solutions, and the like can be employed.

The water is blended in an amount of 0.01 to 10% by mass relative to the total mass of the cosmetic composition. The amount is preferably from 0.1 to 8% by mass, more preferably from 0.1 to 5% by mass. When the amount is less than 0.01% by mass, the desired structure-recovering property is not obtained. Moreover, when the amount exceeds 10% by mass, transparency cannot be maintained and a decrease in viscosity with time is observed and thus the case is not preferred.

In the cosmetic composition of the invention, according to need, further, it is possible to blend various components commonly used in cosmetics, pharmaceuticals, and the like within the range where the effects of the invention are not impaired. Examples thereof include a moisturizer; a hydrocarbon; a higher alcohol; a higher fatty acid; a triglyceride, an ester oil, an animal or vegetable oil and fat, or a silicone other than the components (C) and (E) ; a vitamin; a UV absorber; a water-soluble polymer; an antioxidant; a cationic surfactant; an anionic surfactant; an amphoteric surfactant; a nonionic surfactant other than the component (B); a metal ion sequestrant; ethanol; a thicker; an antiseptic agent; a coloring matter; a pigment; a perfume; and the like.

Moreover, the form of the cosmetic composition of the invention is not particularly limited and can be any of various forms such as a cleansing oil, a hair oil, or a massage oil.

Examples

The following will describe the present invention in further detail with reference to Examples but the invention should not be construed as being limited thereto.

Synthetic Examples of the component (A) according to the invention are shown. The hydroxyl value was measured in accordance with JIS K 1557 1.

[Synthetic Examples]

Synthetic Example 1: Polyoxybutylene (48 mol) polyoxyethylene (88 mol) pentaerythritol ether (Compound 1)

Into an autoclave were charged 45 g of pentaerythritol, 50 g of toluene, and 8.0 g of potassium hydroxide. After the air in the autoclave was replaced by dry nitrogen, 1,292 g of ethylene oxide was added dropwise from a dropping apparatus at 110°C with stirring, followed by stirring for 2 hours. Subsequently, 1,208 g of 1,2-butylene oxide was added dropwise at 110°C, followed by stirring for 2 hours. Thereafter, a reaction product was taken out of the autoclave and neutralized with hydrochloric acid to be a pH of 6 to 7. For removing toluene and water contained, a treatment under reduced pressure was performed at 115°C for 1 hour and finally salts were removed by filtration to obtain 2,345 g of Compound 1. The hydroxyl value was 56.0 mgKOH/g after the reaction with ethylene oxide and 30.0 mgKOH/g after the reaction with 1,2-butylene oxide.

Synthetic Example 2: Polyoxybutylene (45 mol) polyoxyethylene (75 mol) glyceryl (Compound 2)

Into an autoclave were charged 31 g of glycerin and 5.0 g of potassium hydroxide. After the air in the autoclave was replaced by dry nitrogen, 1,100 g of ethylene oxide was added dropwise from a dropping apparatus at 140°C with stirring, followed by stirring for 2 hours. Subsequently, 1,080 g of 1,2-butylene oxide was added dropwise at 140°C, followed by stirring for 2 hours. Thereafter, a reaction product was taken out of the autoclave and neutralized with hydrochloric acid to be a pH of 6 to 7. For removing water contained, a treatment under reduced pressure was performed at 100°C for 1 hour and finally salts were removed by filtration to obtain 2,180 g of polyoxybutylene (45 mol) polyoxyethylene (75 mol) glyceryl ether. The hydroxyl value was 49.5 mgKOH/g after the reaction with ethylene oxide and 25.4 mgKOH/g after the reaction with 1,2-butylene oxide.

The present inventors synthesized polyalkylene glycol derivatives having compositions shown in the following Table 1 in accordance with the above Synthetic Examples 1 and 2. In Table 1, Compounds 1 to 5 are the component (A) of the invention, and Compounds 6 to 10 are comparative control component (A').

[Table 1]

Table 1

| Compound | Z (m) | AO (*1) | EO (a) | AO (k) | EO parts by mass |
|----------|-------|---------|--------|--------|------------------|
| 1 | Pentaerythritol (4) | C4 | 22 | 12 | 52.8 |
| 2 | Glycerin (3) | C4 | 25 | 15 | 50.5 |
| 3 | Pentaerythritol (4) | C4 | 10 | 20 | 23.4 |

(continued)

| Compound | Z (m) | AO (*1) | EO (a) | AO (k) | EO parts by mass |
|----------|-------|---------|--------|--------|------------------|
| 4 | Sorbitol (6) | C8 | 15 | 10 | 34.0 |
| 5 | Diglycerin (4) | C4 | 40 | 10 | 71.0 |
| 6 | Diglycerin (4) | C4 | 0 | 25 | 0 |
| 7 | Glycerin (3) | - | 20 | 0 | 100 |
| 8 | Trehalose (8) | C4 | 40 | 10 | 71.0 |
| 9 | Diethylene glycol (2) | C4 | 45 | 40 | 40.7 |
| 10 | Pentaerythritol (4) | C3 | 22 | 12 | 58.2 |
| (*1) C3 represents a 1,2-oxypropylene group, C4 represents a 1,2-oxybutylene group, and C8 represents a 1,2-oxyoctylene group | | | | | |

[Examples 1 to 10, Comparative Examples 1 to 7]

Cosmetic compositions were prepared with the components and compositions shown in Table 2 and were evaluated for the structure-recovering property, transparency, thermal stability, smoothness when applied to the skin, and fitness with the skin.

<Preparation Method>

After the component (A), the component (B) or (B'), the component (C), and the component (E) were homogeneously dissolved at 70°C, the component (D) was gradually added and the whole was cooled to 25°C with stirring.

<Evaluation Method>

Structure-recovering property:

At 25°C, shear viscosity (Pa·s) against shear velocity (s⁻¹) was measured twice. In this regard, a measuring interval was made 1 minute. A measuring instrument is as follows.
Measuring instrument: Paar Physica MCR-300
Measuring tool: CP 50-2
After it was confirmed that the shear viscosity decreased against the shear velocity at both of the first time and the second time, the structure-recovering property was judged using a recovering ratio of the shear viscosity at a shear velocity of 0.1 (s⁻¹) as an index. "○" was regarded as qualified.
○: Structure-recovering property is observed.
Shear viscosity is recovered by 90% or more at the second time as compared with the case of the first time.
Δ: Poor structure-recovering property is observed.
Shear viscosity is recovered by 70% or more at the second time as compared with the case of the first time.
×: No structure-recovering property is observed.
Shear viscosity is recovered by 50% or less at the second time as compared with the case of the first time.

Transparency:

Appearance was visually confirmed at 25°C.
○: The composition is transparent.
Δ: Fluorescent color is observed.
×: The composition is turbid or white crystals are precipitated.

Thermal stability:

Temperature dependency of loss modulus G" (Pa) at strain 1 (%) was measured. A measuring instrument is as follows.
Measuring instrument: Paar Physica MCR-300

Measuring tool: CP 50-2

○: G" is not decreased even at 40°C or higher.

Δ: A decrease in G" is observed at 35 to 40°C.

×: A decrease in G" is observed at 35°C or lower

Smoothness when applied

The cosmetic composition immediately after preparation was applied to an inner part of the forearm of 20 specialized female panelists under an environment of 25°C and relative humidity of 50%. Evaluation was performed for the smoothness when applied based on the following criteria. An average point of 3.5 or more was regarded as a qualifying criterion.

> 5: Spreadability is very good and an extremely light feeling is exhibited.
> 4: Spreadability is good and a light feeling is exhibited.
> 3: Spreadability is slightly poor and a slightly scratchy feeling is exhibited.
> 2: Spreadability is bad and a heavy feeling is exhibited.
> 1: Spreadability is very bad and a sticky feeling is exhibited.

Fitness with the skin

Following the evaluation for the smoothness when applied, the composition was evaluated for fitness with the skin after the passage of 1 hour under an environment of 25°C and relative humidity of 50% based on the following criteria. An average point of 3.5 or more was regarded as a qualifying criterion.

> 5: A film feeling is good and a feeling of softening the skin is exhibited.
> 4: An appropriate film feeling is observed and a feeling of softening the skin is exhibited.
> 3: A film feeling is slightly strong but a feeling of softening the skin is exhibited.
> 2: A film feeling is slightly strong and a feeling of slightly softening the skin is exhibited.
> 1: A film feeling was strong and a feeling of stiffened skin is exhibited.

Evaluation results thereof are also collectively shown in Table 2.

[Table 2]

Table 2

| | Component | HLB | Lipophilic group | Example (% by mass) | | | | | | | | | | Comparative Example (% by mass) | | | | | | |
|---|---|---|---|---|---|---|---|---|---|---|---|---|---|---|---|---|---|---|---|---|
| | | | | 1 | 2 | 3 | 4 | 5 | 6 | 7 | 8 | | 10 | 1 | 2 | 3 | 4 | 5 | 6 | 7 |
| A | Compound 1 | | | 10.0 | 10.0 | 10.0 | | | 10.0 | 20.0 | 5.0 | | 15.0 | 10.0 | 10.0 | 10.0 | 10.0 | 10.0 | 10.0 | 18.0 |
| | Compound 5 | | | | | | 10.0 | 10.0 | | | | | | | | | | | | |
| B | Sorbitan monooleate | 6.8 | Oleyl group | 5.0 | | | | | 5.0 | | | | 3.0 | | | | | 5.0 | 5.0 | 3.0 |
| | Sorbitan sesquioleate | 4.9 | Oleyl group | | 5.0 | | | | | | 10.0 | | | | | | | | | |
| | Sorbitan monoisostearate | 6.8 | Isostearyl group | | | 5.0 | | | | | | | | | | | | | | |
| | Glyceryl monooleate | 4.2 | Oleyl group | | | | 5.0 | | | | | | | | | | | | | |
| | Diglycerin diisostearate | 3.7 | Isostearyl group | | | | | 5.0 | | 5.0 | | | | | | | | | | |
| B' | Decaglycerin diisostearate | 11.2 | Isostearyl group | | | | | | | | | | | 5.0 | | | | | | |
| | Glycerin monocaprate | 6.8 | Capryl group | | | | | | | | | | | | 5.0 | | | | | |
| | Diglycerin triisostearate | 2.3 | Isostearyl group | | | | | | | | | | | | | 5.0 | | | | |
| | POE (2mol) monooleate | 4.7 | Oleyl group | | | | | | | | | | | | | | 5.0 | | | |

EP 2 732 808 B1

| | | | | | | | | | | | | | | | | | | |
|---|---|---|---|---|---|---|---|---|---|---|---|---|---|---|---|---|---|---|
| C | 2-Ethylhexyl palmitate | | | | 82.0 | 82.0 | | | | | | | | | | | | |
| | Glyceryl tri(2-ethylhexanoate) | 82.0 | 82.0 | 82.3 | | | 27.0 | | 82.5 | | 81.0 | 82.0 | 82.0 | 82.0 | 82.0 | 82.0 | 20.0 | 82.0 |
| | Isononyl isononanoate | | | | | | | 42.0 | | | | | | | | | | |
| E | Dimethicone | | | | | | | 30.0 | | | | | | | | | | |
| | Liquid paraffin | | | | | | 55.0 | | | | | | | | | | 60.0 | |
| D | Water | 3.0 | 3.0 | 2.7 | 3.0 | 3.0 | 3.0 | 3.0 | 2.5 | | 1.0 | 3.0 | 3.0 | 3.0 | 3.0 | 11.0 | 3.0 | 3.0 |
| | (A)/(B) or (A)/(B') mass ratio | 2.0 | 2.0 | 2.0 | 2.0 | 2.0 | 2.0 | 4.0 | 0.5 | | 5.0 | 2.0 | 2.0 | 2.0 | 2.0 | 2.0 | 2.0 | 6.0 |
| | Parts by mass of component (C) in 100 parts by weight of all oily agents | 100 | 100 | 100 | 100 | 100 | 32.9 | 58.3 | 100 | | 100 | 100 | 100 | 100 | 100 | 100 | 25.0 | 100 |
| | Structure-recovering property | ○ | ○ | ○ | ○ | ○ | ○ | ○ | ○ | | ○ | △ | × | ○ | ○ | × | × | ○ |
| | Transparency | ○ | ○ | ○ | ○ | ○ | ○ | ○ | ○ | | ○ | × | ○ | △ | △ | × | × | × |
| | Thermal stability | ○ | ○ | ○ | ○ | ○ | ○ | ○ | ○ | | ○ | ○ | × | △ | △ | × | × | × |
| | Smoothness when applied | 4.0 | 4.1 | 4.1 | 3.9 | 3.8 | 4.2 | 4.5 | 3.7 | | 3.7 | 3.7 | 3.6 | 3.8 | 3.8 | 2.8 | 2.8 | 2.9 |
| | Fitness with skin | 4.0 | 4.2 | 4.1 | 3.8 | 3.9 | 4.3 | 4.2 | 3.8 | | 3.6 | 3.8 | 3.1 | 3.4 | 3.7 | 2.1 | 2.5 | 2.4 |

11

[Examples 11 to 14, Comparative Examples 8 to 14]

Cosmetic compositions were prepared with the components and compositions shown in Table 3 and were evaluated for the structure-recovering property, transparency, thermal stability, smoothness when applied to the skin, and fitness with the skin.

<Preparation Method>

After the component (A) or (A'), the component (B), and the component (C) were homogeneously dissolved at 70°C, the component (D) was gradually added and the whole was cooled to 25°C with stirring.

<Evaluation Method>

Structure-recovering property:

Similarly to Examples 1 to 10, the cosmetic compositions were evaluated for the structure-recovering property, transparency, thermal stability, smoothness when applied to the skin, and fitness with the skin.
Evaluation results thereof are collectively shown in Table 3.

[Table 3]

Table 3

| | Component | Example (% by mass) | | | | Comparative Example (% by mass) | | | | | | |
|---|---|---|---|---|---|---|---|---|---|---|---|---|
| | | 11 | 12 | 13 | 14 | 8 | 9 | 10 | 11 | 12 | 13 | 14 |
| A | Compound 2 | 10.0 | | | | | | | | | | |
| | Compound 3 | | 10.0 | | | | | | | | | |
| | Compound 4 | | | 10.0 | | | | | | | | |
| | Compound 5 | | | | 10.0 | | | | | | | |
| A' | Compound 6 | | | | | 10.0 | | | | | | |
| | Compound 7 | | | | | | 10.0 | | | | | |
| | Compound 8 | | | | | | | 10.0 | | | | |
| | Compound 9 | | | | | | | | 10.0 | | | |
| | Compound 10 | | | | | | | | | 10.0 | | |
| | Dextrin palmitate (*2) | | | | | | | | | | 3.0 | |
| | N-Lauroyl-L-glutamic dibutylamide (*3) | | | | | | | | | | | 1.0 |
| B | Sorbitan monooleate HLB=6.8 lipophilic group: oleyl group | 5.0 | 5.0 | 5.0 | 5.0 | 5.0 | 5.0 | 5.0 | 5.0 | 5.0 | 5.0 | 5.0 |
| C | Glyceryl tri(2-ethylhexanoate) | 82.0 | 82.0 | 82.0 | 82.0 | 82.0 | 82.0 | 82.0 | 82.0 | 82.0 | 91.0 | 93.0 |
| D | Water | 3.0 | 3.0 | 3.0 | 3.0 | 3.0 | 3.0 | 3.0 | 3.0 | 3.0 | 1.0 | 1.0 |
| | (A)/(B) or (A)/(B') mass ratio | 2.0 | 2.0 | 2.0 | 2.0 | 2.0 | 2.0 | 2.0 | 2.0 | 2.0 | 2.0 | 2 |
| | Structure-recovering property | ○ | ○ | ○ | ○ | × | × | ○ | ○ | × | × | × |
| | Transparency | ○ | ○ | ○ | ○ | × | × | Δ | ○ | × | × | × |
| | Thermal stability | ○ | ○ | ○ | ○ | × | × | Δ | Δ | × | ○ | ○ |
| | Smoothness when applied | 3.8 | 4.1 | 3.8 | 3.9 | 2.3 | 1.8 | 2.1 | 3.3 | 1.9 | 3.2 | 3.1 |
| | Fitness with skin | 3.7 | 4.0 | 4.0 | 4.0 | 2.1 | 1.7 | 1.9 | 3.2 | 1.6 | 2.1 | 2.4 |

*2 Rheopearl KL2 manufactured by Chiba Flour Milling Co., Ltd.
*3 GP-1 manufactured by Ajinomoto Co., Inc.

The following will exemplify referential formulation of the cosmetic composition of the invention. In all Formulation Examples, "structure-recovering property", "transparency", "thermal stability", "smoothness when applied", and "fitness with the skin" were good.

<Formulation Example 1: Hair Oil>

| | |
|---|---|
| (A) Compound 1 | 17.0% by mass |
| (B) Diglycerin monooleate (HLB 6.8) | 5.0% by mass |
| (B) Diglycerin diisostearate (HLB 3.7) | 5.0% by mass |
| (C) Glyceride of tri(caprylate/caprate) | 20.0% by mass |
| (C) Isononanyl isononanate | 10.0% by mass |
| (C) Olive oil | 5.0% by mass |
| (C) Camellia oil | 3.0% by mass |
| (E) Hydrogenated polyisobutene | 20.0% by mass |
| (E) Cyclomechicone | 10.0% by mass |
| (D) Water | 3.5% by mass |
| Vitamin E acetate | 0.2% by mass |
| Polyoxyethylene (10 mol) methylglucoside | 0.5% by mass |
| Glycerin | 0.2% by mass |
| Antiseptic agent | proper quantity |
| Perfume | proper quantity |

<Preparation Method>

After the components other than the component (D) were homogeneously dissolved at 70°C, the component (D) was gradually added and the whole was cooled to 25°C with stirring.

<Formulation Example 2: Massage Oil>

| | |
|---|---|
| (A) Compound 1 | 5.0% by mass |
| (A) Compound 5 | 10.0% by mass |
| (B) Sorbitan sesquiisostearate (HLB 4.9) | 3.0% by mass |
| (B) Glycerin monoisostearate (HLB 4.2) | 2.0% by mass |
| (C) Isopropyl palmitate | 15.0% by mass |
| (C) Ethyl oleate | 10.0% by mass |
| (C) Safflower oil | 15.0% by mass |
| (C) Castor oil | 3.0% by mass |
| (E) Liquid paraffin | 15.0% by mass |
| (E) Squalane | 15.0% by mass |
| (D) Water | 3.5% by mass |
| Dipropylene glycol | 1.0% by mass |
| 1,2-Octylene glycol | 0.5% by mass |
| Vitamin E | 0.5% by mass |
| Antiseptic agent | proper quantity |
| Perfume | proper quantity |

<Preparation Method>

After the components other than the component (D) were homogeneously dissolved at 70°C, the component (D) was gradually added and the whole was cooled to 25°C with stirring.

<Formulation Example 3: Cleansing Oil>

| | |
|---|---|
| (A) Compound 1 | 15.0% by mass |
| (B) Diglycerin monoisostearate (HLB 3.2) | 5.0% by mass |
| (B) Sorbitan monooleate (HLB 6.8) | 3.0% by mass |
| (C) 2-Ethylhexyl palmitate | 20.0% by mass |
| (C) Glyceride of tri(2-ethylhexanoate) | 25.0% by mass |
| (C) Safflower oil | 15.0% by mass |
| (E) Hydrogenated polyisobutene | 13.0% by mass |
| (D) Water | 3.0% by mass |
| 1,3-Butylene glycol | 0.2% by mass |
| 1,2-Hexylene glycol | 0.2% by mass |
| Vitamin E | 0.3% by mass |
| Antiseptic agent | proper quantity |
| Perfume | proper quantity |

<Preparation Method>

After the components other than the component (D) were homogeneously dissolved at 70°C, the component (D) was gradually added and the whole was cooled to 25°C with stirring.

Claims

1. A cosmetic composition comprising the following components (A) to (D), wherein
the component (A) is from 0.1 to 30% by mass,
the component (B) is from 0.1 to 30% by mass,
the component (C) is from 50 to 95% by mass, wherein a part of component (C) can be replaced by the component (E) and an amount of the component (C) is 30 parts by mass or more relative to 100 parts by mass of a total amount of the component (C) and the component (E), and
the component (D) is from 0.01 to 10% by mass, each relative to the total mass of the cosmetic composition, wherein the amounts of the components (A) to (D) add up to 100% by mass, and a mass ratio of the component (A) to the component (B), (A) / (B) is from 1/5 to 5/1:

(A) a polyalkylene glycol derivative represented by the following formula (I):

$$Z\text{-}\{O\text{-}(EO)_a\text{-}(AO)_k\text{-}H\}_m \qquad (I)$$

wherein Z is a residual group resulting from removal of hydroxyl groups from a polyhydric alcohol having 3 to 6 hydroxyl groups, m is from 3 to 6, EO is an oxyethylene group, AO is an oxyalkylene group having 4 to 8 carbon atoms, and EO and AO are bonded in a block form;
a represents an average addition mole number of EO and k represents an average addition mole number of AO, and a is from 1 to 50 and k is from 1 to 50; and a mass ratio of EO is from 10 to 75 parts by mass relative to 100 parts by mass of a total amount of EO and AO,

(B) a nonionic surfactant having a hydroxyl group and a hydrocarbon group having 12 to 22 carbon atoms and exhibiting an HLB of 3 to 8,
(C) one or two or more oily agents selected from ester oils and triglycerides which are liquid at 25°C, and
(D) water,
(E) one or two or more oily agents selected from hydrocarbon oils and silicone oils which are liquid at 25°C,

wherein the HLB can be calculated form the following formula (1) or (2) :

(1) glucide-type nonionic surfactant

$$HLB = (\text{sum of inorganicity value}/\text{sum of organicity value}) \times 10$$

(2) Polyglycerin-type or sorbitan nonionic surfactant

$$HLB = 20(1-S/A)$$

S: saponification value of ester, A: acid value of fatty acid.

**2.** The cosmetic composition according to claim 1, wherein AO is a 1,2-oxybutylene group.

**3.** The cosmetic composition according to claim 1 to 2, wherein the component (B) is a nonionic surfactant having a branched or unsaturated hydrocarbon group having 12 to 22 carbon atoms.

**Patentansprüche**

**1.** Kosmetikzusammensetzung mit den folgenden Komponenten (A) bis (D), worin die Menge der Komponente (A) von 0,1 bis 30 Masse-% ist, die Menge der Komponente (B) von 0,1 bis 30 Masse-% ist, die Menge der Komponente (C) von 50 bis 95 Masse-% ist, worin ein Teil der Komponente (C) durch die Komponente (E) ersetzt sein kann und eine Menge der Komponente (C) 30 Massenteile oder mehr in Bezug auf 100 Massenteile einer Gesamtmenge der Komponente (C) und der Komponente (E) ist, und die Komponente (D) von 0,01 bis 10 Masse-% ist, jeweils in Bezug auf die Gesamtmasse der Kosmetikzusammensetzung, worin die Mengen der Komponenten (A) bis (D) sich auf 100 Masse-% addieren und ein Massenverhältnis der Komponente (A) zu der Komponente (B), (A)/(B), von 1/5 bis 5/1 ist:

(A) ein Polyalkylenglykolderivat mit der folgenden Formel (I)

$$Z\text{-}\{O\text{-}(EO)_a\text{-}(AO)_k\text{-}H\}_m \qquad (I)$$

worin Z eine Restgruppe ist, die von der Entfernung von Hydroxylgruppen von einem mehrwertigen Alkohol mit 3 bis 6 Hydroxylgruppen resultiert, m von 3 bis 6 ist, EO eine Oxyethylengruppe ist, AO eine Oxyalkylengruppe mit 4 bis 8 Kohlenstoffatomen ist und EO und AO in einer Blockform gebunden sind; a eine durchschnittliche Additionsmolzahl von EO ist und k eine durchschnittliche Additionsmolzahl von AO ist, und a von 1 bis 50 ist und k von 1 bis 50 ist; und ein Massenverhältnis von EO von 10 bis 75 Massenteilen in Bezug auf 100 Massenteile einer gesamten Menge von EO und AO ist;

(B) ein nicht-ionisches Tensid mit einer Hydroxylgruppe und einer Kohlenwasserstoffgruppe mit 12 bis 22 Kohlenstoffatomen und mit einem HLB von 3 bis 8,
(C) ein oder zwei oder mehrere ölige Mittel, ausgewählt aus Esterölen und Triglyceriden, die bei 25°C flüssig sind, und
(D) Wasser,
(E) ein oder zwei oder mehrere ölige Mittel, ausgewählt aus Kohlenwasserstoffölen und Silikonölen, die bei 25°C flüssig sind,

worin der HLB durch folgende Formel (1) oder (2) berechnet werden kann:

(1) nicht-ionisches Tensid vom Glucid-Typ

```
HLB = (Summe des Anorganizitätswertes/Summe des
Organizitätswertes) × 10
```

(2) Polyglycerin-Typ- oder Sorbitan-nicht-ionisches Tensid

```
HLB = 20(1-S/A)
```

S: Verseifungsgrad von Ester, A: Säurewert von Fettsäure.

2. Kosmetikzusammensetzung gemäß Anspruch 1, worin AO eine 1,2-Oxybutylengruppe ist.

3. Kosmetikzusammensetzung gemäß Anspruch 1 bis 2, worin die Komponente (B) ein nicht-ionisches Tensid mit einer verzweigten oder ungesättigten Kohlenwasserstoffgruppe mit 12 bis 22 Kohlenstoffatomen ist.

**Revendications**

1. Composition cosmétique comprenant les constituants (A) à (D) suivants, dans laquelle
le constituant (A) est de 0,1 à 30 % en masse,
le constituant (B) est de 0,1 à 30 % en masse,
le constituant (C) est de 50 à 95 % en masse, dans laquelle une partie du constituant (C) peut être remplacée par le constituant (E) et une quantité du constituant (C) est de 30 parties en masse ou supérieure par rapport à 100 parties en masse d'une quantité totale du constituant (C) et du constituant (E), et
le constituant (D) est de 0,01 à 10 % en masse, chacun par rapport à la masse totale de la composition cosmétique, dans laquelle les quantités des constituants (A) à (D) s'additionnent jusqu'à 100 % en masse, et un rapport massique du constituant (A) au constituant (B), (A)/(B) est de 1/5 à 5/1 :

(A) un dérivé de polyalkylène glycol représenté par la formule (I) suivante :

$$Z\text{-}\{O\text{-}(EO)_a\text{-}(AO)_k\text{-}H\}_m \qquad (I)$$

dans laquelle Z est un groupe résiduel résultant de l'élimination de groupes hydroxyle d'un alcool polyvalent ayant de 3 à 6 groupes hydroxyle, m est de 3 à 6, EO est un groupe oxyéthylène, AO est un groupe oxyalkylène ayant de 4 à 8 atomes de carbone, et EO et AO sont liés dans une forme de séquence ;
a représente un nombre moyen de mole d'addition de EO et k représente un nombre moyen de mole d'addition de AO, et a est de 1 à 50 et k est de 1 à 50 ; et un rapport massique de EO est de 10 à 75 parties en masse par rapport à 100 parties en masse d'une quantité totale de EO et AO,

(B) un tensioactif non-ionique ayant un groupe hydroxyle et un groupe hydrocarboné ayant de 12 à 22 atomes de carbone et présentant un HLB de 3 à 8,
(C) un ou deux agents huileux ou plus choisis parmi des huiles d'esters et des triglycérides qui sont liquides à 25°C, et
(D) de l'eau,
(E) un ou deux agents huileux ou plus choisis parmi des huiles hydrocarbonées et des huiles de silicone qui sont liquides à 25°C,

dans laquelle le HLB peut être calculé par la formule (1) ou (2) suivante :

(1) tensioactif non-ionique de type glucide

HLB = (somme de valeur d'inorganicité/somme de valeur d'organicité) x 10

(2) tensioactif non-ionique de type polyglycérine ou sorbitane

$$HLB = 20 \ (1 - S/A)$$

S : indice de saponification d'ester, A : indice acide d'acide gras.

2. Composition cosmétique selon la revendication 1, dans laquelle AO est un groupe 1,2-oxybutylène.

3. Composition cosmétique selon la revendication 1 à 2, dans laquelle le constituant (B) est un tensioactif non-ionique présentant un groupe hydrocarboné ramifié ou insaturé ayant de 12 à 22 atomes de carbone.

# FIG. 1

# FIG. 2

SHEAR
VISCOSITY (Pa·s)

SHEAR VELOCITY (1/s)

# FIG. 3

LOSS
MODULUS G"

TEMPERATURE (°C)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002003340 A
- JP 2002316971 A
- JP 2008019239 A
- JP 2010024159 A
- US 20100190864 A1
- US 20090163616 A1

### Non-patent literature cited in the description

- Shin-pan Kaimen Kasseizai Handobukku. Surfactant Handbook. Kougaku Tosho K.K,
- **KOUDA.** Yuuki Gainen-zu - Kiso to Ouyo (Organic Conceptual Diagram - Base and Application. Sankyo Publishing Co., Ltd,
- **YAGUCHI.** Yuuki Ginen-zu niyoru Nyuuka Syohou Sekkei (Emulsification Formulation Design by Organic Conceptual Diagram. Nihon Emulsion Co., Ltd,